# EUROPEAN PATENT SPECIFICATION

(11) **EP 3 922 921 B1**
(45) Date of publication and mention of the grant of the patent: **15.07.2026**
(21) Application number: 20752309.3
(22) Date of filing: 07.02.2020
(51) Int. Cl.: F25B 1/00, C09K 5/04, C07C 17/383

(54) **COMPOSITION CONTAINING TRANS-1,2-DIFLUOROETHYLENE (HFO-1132(E)) AND 1,1,1-TRIFLUOROETHANE (HFC-143A) AND METHOD FOR SEPARATING HFO-1132(E) AND HFC-143A FROM COMPOSITION CONTAINING HFO-1132(E) AND HFC-143A**
ZUSAMMENSETZUNG MIT TRANS-1,2-DIFLUOROETHYLEN (HFO-1132(E)) UND 1,1,1-TRIFLUOROETHAN (HFC-143A) UND VERFAHREN ZUR TRENNUNG VON HFO-1132(E) UND HFC-143A AUS EINER ZUSAMMENSETZUNG MIT HFO-1132(E) UND HFC-143A
COMPOSITION CONTENANT DU TRANS-1,2-DIFLUOROÉTHYLÈNE (HFO-1132(E)) ET DU 1,1,1-TRIFLUOROÉTHANE (HFC-143A) ET PROCÉDÉ PERMETTANT DE SÉPARER LE HFO -1132(E) ET LE HFC-143A D'UNE COMPOSITION CONTENANT LE HFO-1132(E) ET LE HFC-143A

(30) Priority: 07.02.2019 JP 2019020994
(43) Date of publication of application: 15.12.2021
(73) Proprietor: DAIKIN INDUSTRIES, LTD., Osaka-Shi, Osaka 530-0001 (JP)
(72) Inventor: TAKAHASHI, Kazuhiro, Osaka-shi, Osaka 5300001 (JP); NAKAUE, Tsubasa, Osaka-shi, Osaka 5300001 (JP); CHAKI, Takehiro, Osaka-shi, Osaka 5300001 (JP); USUI, Takashi, Osaka-shi, Osaka 5300001 (JP); KUSHIDA, Megumi, Osaka-shi, Osaka 5300001 (JP)
(74) Representative: Hoffmann Eitle
(86) International application number: PCT/JP2020/004919
(87) International publication number: WO 2020/162617

(56) References cited:
- JP-A- 2012 508 306
- JP-A- 2015 229 768
- JP-A- 2016 130 236
- US-A1- 2011 253 927

## Description

### Technical Field

The present disclosure relates to the use of a composition comprising HFO-1132(E) and HFC-143a as a replacement for R410A.

### Background Art

Refrigerants containing 1,2-difluoroethylene in *trans-*(E) form and *cis-*(Z) form are very promising as alternatives to heat-actuated media such as refrigerants used in air conditioners, for which R-410A, HFC-32, HFC-134a, etc. have been used thus far (PTL 1).

The E-1,2-difluoroethylene obtained by this method contains HFC-143a as a byproduct.

At 0°C and 626 kPa, 11 wt% HFO-1132(E) and 89 wt% HFC-143a are known to form an azeotropic composition (PTL 2).

### Citation List

### Patent Literature

PTL 1: WO2012/157765A
PTL 2: US Patent Publication No. 2011/0253927

### Summary of Invention

### Technical Problem

An object of the present disclosure is to provide a use of an azeotropic or azeotrope-like composition comprising HFO-1132(E) and HFC-143a.

### Solution to Problem

The present invention is defined by the claims.

### Advantageous Effects of Invention

The present disclosure provides a use of an azeotropic or azeotrope-like composition comprising HFO-1132(E) and HFC-143a as a replacement for R410A.

### Brief Description of Drawings

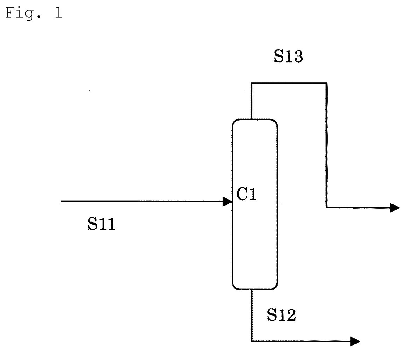

Fig. 1 is a drawing illustrating an example of the process for separating HFO-1132(E) and HFC-143.

### Description of Embodiments

### Definition of Terms

In the present specification, the term "refrigerant" includes at least compounds that are specified in ISO 817 (International Organization for Standardization), and that are given a refrigerant number (ASHRAE number) representing the type of refrigerant with "R" at the beginning; and further includes refrigerants that have properties equivalent to those of such refrigerants, even though a refrigerant number is not yet given. Refrigerants are broadly divided into fluorocarbon compounds and non-fluorocarbon compounds, in terms of the structure of the compounds. Fluorocarbon compounds include chlorofluorocarbons (CFC), hydrochlorofluorocarbons (HCFC), and hydrofluorocarbons (HFC). Non-fluorocarbon compounds include propane (R290), propylene (R1270), butane (R600), isobutane (R600a), carbon dioxide (R744), ammonia (R717), and the like.

In the present specification, the phrase "composition comprising a refrigerant" at least includes (1) a refrigerant itself (including a mixture of refrigerants); (2) a composition that further comprises other components, and that can be mixed with at least a refrigeration oil to obtain a working fluid for a refrigerating machine; and (3) a working fluid for a refrigerating machine containing a refrigeration oil. In the present specification, of these three embodiments, the composition (2) is referred to as a "refrigerant composition" so as to distinguish it from a refrigerant itself (including a mixture of refrigerants). Further, the working fluid for a refrigerating machine (3) is referred to as a "refrigeration oil-containing working fluid" so as to distinguish it from the "refrigerant composition."

In the present specification, when the term "alternative" is used in a context in which the first refrigerant is replaced with the second refrigerant, the first type of "alternative" means that equipment designed for operation using the first refrigerant can be operated using the second refrigerant under optimum conditions, optionally with changes of only a few parts (at least one of the following: refrigeration oil, gasket, packing, expansion valve, dryer, and other parts) and equipment adjustment. In other words, this type of alternative means that the same equipment is operated with an alternative refrigerant. Embodiments of this type of "alternative" include "drop-in alternative," "nearly drop-in alternative," and "retrofit," in the order in which the extent of changes and adjustment necessary for replacing the first refrigerant with the second refrigerant is smaller.

The term "alternative" also includes a second type of "alternative," which means that equipment designed for operation using the second refrigerant is operated for the same use as the existing use with the first refrigerant by using the second refrigerant. This type of alternative means that the same use is achieved with an alternative refrigerant.

In the present specification, the term "refrigerating machine" refers to machines in general that draw heat from an object or space to make its temperature lower than the temperature of ambient air, and maintain the low temperature. In other words, refrigerating machines refer to conversion machines that gain energy from the outside to do work, and that perform energy conversion, in order to transfer heat from where the temperature is lower to where the temperature is higher.

In the present specification, the term "azeotrope-like composition" refers to a composition that can be handled in substantially the same manner as azeotropic compositions, and specifically means a composition composed of two or more substances that behave substantially as a single substance with a constant boiling point or substantially a constant boiling point.

More specifically, the term "azeotrope-like composition" refers to a composition whose vapor generated by evaporating or distilling the composition in liquid form has a formulation substantially unchanged from the formulation of the liquid. In other words, an azeotrope-like composition can be boiled, distilled, and refluxed without a substantial compositional change.

In the present specification, whether a composition is azeotrope-like is determined based on whether the bubble-point vapor pressure of the composition and the dew-point vapor pressure of the composition at a specific temperature are substantially the same. More specifically, a composition having a difference between the dew-point pressure and the bubble-point pressure of 3 percent or less (based on the bubble-point pressure) is determined to be an azeotrope-like composition.

The present invention relates to a use of a composition comprising a refrigerant, said use being fully characterized by claim 1. The refrigerant employed in this use is herein further discussed. This also pertains to the refrigerant composition discussed below.

### 1. Refrigerant

### 1.1 Refrigerant Component

The refrigerant used in the present disclosure is a mixed refrigerant comprising trans-1,2-difluoroethylene (HFO-1132(E)) and 1,1,1-trifluoroethane (HFC-143a).

The refrigerant used in the present disclosure is an azeotropic or azeotrope-like composition.

In the refrigerant used in the present disclosure, the proportion of HFO-1132(E) is 80 mass% or more and less than 100 mass%, and the proportion of HFC-143a is more than 0 mass% and 20 mass% or less, based on the total amount of HFO-1132 (E) and HFC-143a. The refrigerant used in the present disclosure within this range of the formulation has a COP of 97% or more relative to R410A and a refrigerating capacity of 94% or more relative to R410A, and has a GWP of 1500 or less based on the AR5 (the IPCC Fifth Assessment Report).

In the refrigerant used in the present disclosure, the proportion of HFO-1132 (E) is more preferably 90 mass% or more and less than 100 mass%, and the proportion of HFC-143a is more preferably more than 0 mass% and 10 mass% or less, based on the total amount of HFO-1132(E) and HFC-143. The refrigerant used in the present disclosure within this range of formulation has a COP of 98% or more relative to R-410A, exhibiting a performance comparable to R-410A, and a GWP of 750 or less based on the AR5 (the IPCC Fifth Assessment Report).

The refrigerant used in the present disclosure may further comprises an additional refrigerant. The additional refrigerant is not limited, and can be selected from a wide range of refrigerants. The additional refrigerant is, for example, at least one refrigerant selected from the group consisting of 1,1,2-trifluoroethylene (HFO-1123), 1,1-difluoromethane, fluoroethane (HFC-152a), 1,1,2-trifluoroethane (HFC-143), 2-chloro-1,1,1-trifluoroethane (CFC-133a), 1-chloro-1,1,2-trifluoroethane (HCFC-133b), 2,2-dichloro-1,1,1-trifluoroethane (HCFC-123), and 1,2-difluoroethane (HFC-152).

The proportion of the total amount of the additional refrigerant in the refrigerant used in the present disclosure is preferably more than 0 mass% and 1 mass% or less, more preferably more than 0 mass% and 0.5 mass% or less, and still more preferably more than 0 mass% and 0.1 mass% or less based on the total amount of HFO-1132(E), HFC-143a, and the total amount of the additional refrigerant. The refrigerant used in the present disclosure within this range of formulation is likely to become azeotropic or azeotrope-like.

### 1.2. Use

The refrigerant used in the present disclosure can be preferably used as a working fluid in a refrigerating machine.

The refrigerant used in the present disclosure is used as an alternative refrigerant for R410A.

### 2. Refrigerant Composition

The refrigerant composition used in the present disclosure comprises at least the refrigerant used in the present disclosure, and is used for the same use as the refrigerant used in the present disclosure. Moreover, the refrigerant composition can be further mixed with at least a refrigeration oil to thereby obtain a working fluid for a refrigerating machine.

The refrigerant composition used in the present disclosure further comprises at least one other component in addition to the refrigerant used in the present disclosure. The refrigerant composition may comprise at least one of the following other components, if necessary. As described above, when the refrigerant composition is used as a working fluid in a refrigerating machine, it is generally used as a mixture with at least a refrigeration oil. Therefore, it is preferable that the refrigerant composition does not substantially comprise a refrigeration oil. Specifically, the content of the refrigeration oil in the refrigerant composition is preferably 0 to 1 mass%, and more preferably 0 to 0.1 mass% based on the entire refrigerant composition.

### 2.1. Water

The refrigerant composition used in the present disclosure may contain a small amount of water. The water content in the refrigerant composition is preferably 0.1 mass% or less based on the entire refrigerant. A small amount of water contained in the refrigerant composition stabilizes double bonds in the molecules of unsaturated fluorocarbon compounds that can be present in the refrigerant, and makes it less likely that the unsaturated fluorocarbon compounds will be oxidized, thus increasing the stability of the refrigerant composition.

### 2.2. Tracer

A tracer is added to the refrigerant composition used in the present disclosure at a detectable concentration such that when the refrigerant composition has been diluted, contaminated, or undergone other changes, the tracer can trace the changes.

The refrigerant composition according to the present disclosure may comprise a single tracer, or two or more tracers.

The tracer is not limited, and can be suitably selected from commonly used tracers.

Examples of tracers include hydrofluorocarbons, hydrochlorofluorocarbons, chlorofluorocarbons, hydrochlorocarbons, fluorocarbons, deuterated hydrocarbons, deuterated hydrofluorocarbons, perfluorocarbons, fluoroethers, brominated compounds, iodinated compounds, alcohols, aldehydes, ketones, and nitrous oxide (N₂O). The tracer is particularly preferably a hydrofluorocarbon, a hydrochlorofluorocarbon, a chlorofluorocarbon, a hydrochlorocarbon, a fluorocarbon, or a fluoroether.

Specifically, the following compounds are preferable as the tracer.
FC-14 (tetrafluoromethane, CF₄)
HCC-40 (chloromethane, CH₃Cl)
HFC-23 (trifluoromethane, CHF₃)
HFC-41 (fluoromethane, CH₃Cl)
HFC-125 (pentafluoroethane, CF₃CHF₂)
HFC-134a (1,1,1,2-tetrafluoroethane, CF₃CH₂F)
HFC-134 (1,1,2,2-tetrafluoroethane, CHF₂CHF₂)
HFC-143a (1,1,1-trifluoroethane, CF₃CH₃)
HFC-143 (1,1,2-trifluoroethane, CHF₂CH₂F)
HFC-152a (1,1-difluoroethane, CHF₂CH₃)
HFC-152 (1,2-difluoroethane, CH₂FCH₂F)
HFC-161 (fluoroethane, CH₃CH₂F)
HFC-245fa (1, 1, 1, 3, 3-pentafluoropropane, CF₃CH₂CHF₂)
HFC-236fa (1,1,1,3,3,3-hexafluoropropane, CF₃CH₂CF₃)
HFC-236ea (1,1,1,2,3,3-hexafluoropropane, CF₃CHFCHF₂)
HFC-227ea (1,1,1,2,3,3,3-heptafluoropropane, CF₃CHFCF₃)
HCFC-22 (chlorodifluoromethane, CHClF₂)
HCFC-31 (chlorofluoromethane, CH₂ClF)
CFC-1113 (chlorotrifluoroethylene, CF₂=CClF)
HFE-125 (trifluoromethyl-difluoromethyl ether, CF₃OCHF₂)
HFE-134a (trifluoromethyl-fluoromethyl ether, CF₃OCH₂F)
HFE-143a (trifluoromethyl-methyl ether, CF₃OCH₃)
HFE-227ea (trifluoromethyl-tetrafluoroethyl ether, CF₃OCHFCF₃)
HFE-236fa (trifluoromethyl-trifluoroethyl ether, CF₃OCH₂CF₃)

The refrigerant composition used in the present disclosure may comprise a tracer in a total amount of about 10 parts per million (ppm) to about 1000 ppm based on the entire refrigerant composition. The refrigerant composition used the present disclosure may comprise a tracer in a total amount of preferably about 30 ppm to about 500 ppm, and more preferably about 50 ppm to about 300 ppm based on the entire refrigerant composition.

### 2.3. Ultraviolet Fluorescent Dye

The refrigerant composition used in the present disclosure may comprise a single ultraviolet fluorescent dye, or two or more ultraviolet fluorescent dyes.

The ultraviolet fluorescent dye is not limited, and can be suitably selected from commonly used ultraviolet fluorescent dyes.

Examples of ultraviolet fluorescent dyes include naphthalimide, coumarin, anthracene, phenanthrene, xanthene, thioxanthene, naphthoxanthene, fluorescein, and derivatives thereof. The ultraviolet fluorescent dye is particularly preferably either naphthalimide or coumarin, or both.

### 2.4. Stabilizer

The refrigerant composition used in the present disclosure may comprise a single stabilizer, or two or more stabilizers.

The stabilizer is not limited, and can be suitably selected from commonly used stabilizers.

Examples of stabilizers include nitro compounds, ethers, and amines.

Examples of nitro compounds include aliphatic nitro compounds, such as nitromethane and nitroethane; and aromatic nitro compounds, such as nitrobenzene and nitrostyrene.

Examples of ethers include 1,4-dioxane.

Examples of amines include 2,2,3,3,3-pentafluoropropylamine and diphenylamine.

Examples of stabilizers also include butylhydroxyxylene and benzotriazole.

The content of the stabilizer is not limited. Generally, the content of the stabilizer is preferably 0.01 to 5 mass%, and more preferably 0.05 to 2 mass%, based on the entire refrigerant.

### 2.5. Polymerization Inhibitor

The refrigerant composition used in the present disclosure may comprise a single polymerization inhibitor, or two or more polymerization inhibitors.

The polymerization inhibitor is not limited, and can be suitably selected from commonly used polymerization inhibitors.

Examples of polymerization inhibitors include 4-methoxy-1-naphthol, hydroquinone, hydroquinone methyl ether, dimethyl-t-butylphenol, 2,6-di-tert-butyl-p-cresol, and benzotriazole.

The content of the polymerization inhibitor is not limited. Generally, the content of the polymerization inhibitor is preferably 0.01 to 5 mass%, and more preferably 0.05 to 2 mass%, based on the entire refrigerant.

### 3. Refrigeration Oil-Containing Working Fluid

The refrigeration oil-containing working fluid used in the present disclosure comprises at least the refrigerant or refrigerant composition used in the present disclosure and a refrigeration oil, for use as a working fluid in a refrigerating machine. Specifically, the refrigeration oil-containing working fluid according to the present disclosure is obtained by mixing a refrigeration oil used in a compressor of a refrigerating machine with the refrigerant or the refrigerant composition. The refrigeration oil-containing working fluid generally comprises 10 to 50 mass% of refrigeration oil.

### 3.1. Refrigeration Oil

The refrigeration oil-containing working fluid used in the present disclosure may comprise a single refrigeration oil, or two or more refrigeration oils.

The refrigeration oil is not limited, and can be suitably selected from commonly used refrigeration oils. In this case, refrigeration oils that are superior in the action of increasing the miscibility with the mixture and the stability of the mixture, for example, are suitably selected as necessary.

The base oil of the refrigeration oil is preferably, for example, at least one member selected from the group consisting of polyalkylene glycols (PAG), polyol esters (POE), and polyvinyl ethers (PVE).

The refrigeration oil may further contain additives in addition to the base oil. The additive may be at least one member selected from the group consisting of antioxidants, extreme-pressure agents, acid scavengers, oxygen scavengers, copper deactivators, rust inhibitors, oil agents, and antifoaming agents.

A refrigeration oil with a kinematic viscosity of 5 to 400 cSt at 40°C is preferable from the standpoint of lubrication.

The refrigeration oil-containing working fluid used in the present disclosure may further optionally contain at least one additive. Examples of additives include the compatibilizing agents described below.

### 3.2. Compatibilizing Agent

The refrigeration oil-containing working fluid used in the present disclosure may comprise a single compatibilizing agent, or two or more compatibilizing agents.

The compatibilizing agent is not limited, and can be suitably selected from commonly used compatibilizing agents.

Examples of compatibilizing agents include polyoxyalkylene glycol ethers, amides, nitriles, ketones, chlorocarbons, esters, lactones, aryl ethers, fluoroethers, and 1,1,1-trifluoroalkanes. The compatibilizing agent is particularly preferably a polyoxyalkylene glycol ether.

### 4. Method for Operating Refrigerating Machine

The method for operating a refrigerating machine according to the present disclosure is a method for operating a refrigerating machine using the refrigerant discussed above.

Specifically, the method for operating a refrigerating machine according to the present disclosure comprises circulating the refrigerant discussed above in a refrigerating machine.

### 5. Separation Method (Reference embodiment)

The separation method forming a reference embodiment of the present disclosure is a method for separating HFO-1132(E) and HFC-143a from a composition comprising HFO-1132 (E) and HFC-143a, and the method comprises subjecting the composition to azeotropic distillation to separate an azeotropic or azeotrope-like composition comprising HFO-1132(E) and HFC-143a. The separation method is not claimed but may be useful for understanding the present invention.

HFC-143a has a boiling point of -53°C, which is close to the boiling point of HFO-1132(E). Additionally, HFC-143a was found to form an azeotropic composition or azeotrope-like composition together with HFO-1132(E). Thus, it is difficult to separate HFC-143a from HFO-1132 (E) by typical distillation. The separation method brings about an effect such that HFC-143a can be effectively separated from HFO-1132 (E) by subjecting a composition containing HFO-1132 (E) and HFC-143a to azeotropic distillation.

Azeotropic distillation refers to a method by which one or more azeotropic compositions or azeotrope-like compositions are separated by a distillation column, which is operated under conditions in which such compositions are separable.

In azeotropic distillation, the starting composition may be not only a composition that consists of two or more components that form an azeotropic or azeotrope-like composition, but also a composition that contains an additional component in addition to two or more components that form an azeotropic or azeotrope-like composition. In the latter case, azeotropic distillation may be performed after one or more components of a starting composition and the components that form an azeotropic or azeotrope-like composition are added to the starting composition. The use of azeotropic distillation makes it easier to separate a target product.

In the present disclosure, the target product is separated by performing azeotropic distillation by using the change with temperature in compositional region in which an azeotropic or azeotrope-like composition forms.

### Examples

The following describes a reference method for separating a composition formed only of HFO-1132(E) and HFC-143a, and a refrigeration cycle using the method with reference to the Examples.

### Reference Example 1

Table 1 illustrates the calculation results of the vapor-liquid equilibrium of a composition formed only of HFO-1132(E) and HFC-143a.

**Table 1**

| Temperature (°C) | | Pressure (MPa) | Liquid Phase (HFO-1132 (E)) | Gas Phase (HFO-1132 (E)) |
|---|---|---|---|---|
| 20 | | 1.10526 | 0 | 0 |
| 20 | | 1.22568 | 0.1 | 0.16 |
| 20 | | 1.29148 | 0.2 | 0.26 |
| 20 | | 1.33297 | 0.3 | 0.35 |
| 20 | | 1.36114 | 0.4 | 0.44 |
| 20 | | 1.38107 | 0.5 | 0.53 |
| 20 | | 1.39603 | 0.6 | 0.62 |
| 20 | | 1.40852 | 0.7 | 0.71 |
| 20 | | 1.42062 | 0.8 | 0.81 |
| 20 | | 1.42703 | 0.85 | 0.86 |
| 20 | | 1.43387 | 0.9 | 0.91 |
| 20 | | 1.44129 | 0.95 | 0.95 |
| 20 | | 1.44931 | 1 | 1 |
| 40 | | 1.83213 | 0 | 0 |
| 40 | | 2.03698 | 0.1 | 0.15 |
| 40 | | 2.14279 | 0.2 | 0.25 |
| 40 | | 2.20519 | 0.3 | 0.34 |
| 40 | | 2.24447 | 0.4 | 0.43 |
| 40 | | 2.27096 | 0.5 | 0.52 |
| 40 | | 2.29177 | 0.6 | 0.61 |
| 40 | | 2.31223 | 0.7 | 0.71 |
| 40 | | 2.33621 | 0.8 | 0.81 |
| 40 | | 2.35028 | 0.85 | 0.86 |
| 40 | | 2.36599 | 0.9 | 0.91 |
| 40 | | 2.38342 | 0.95 | 0.96 |
| 40 | | 2.4026 | 1 | 1 |

### Example 2

Table 2 illustrates the calculation results of refrigeration cycle on the composition formed only of HFO-1132(E) and HFC-143a. The calculation conditions are as follows.
Evaporating temperature: 5°C
Condensation temperature: 45°C
Superheating temperature: 5°C
Subcooling temperature: 5°C
Compressor efficiency: 0.7

**Table 2**

| Item | Unit | Formulation | | | | | | |
|---|---|---|---|---|---|---|---|---|
| HFO-1132E | mass% | 1 | 10 | 30 | 50 | 70 | 90 | 99 |
| HFC-143a | mass% | 99 | 90 | 70 | 50 | 30 | 10 | 1 |
| GWP | | 4425.3 | 4023.1 | 3129.3 | 2235.5 | 1341.7 | 447.9 | 45.7 |
| COP | | 3.75 | 3.71 | 3.64 | 3.64 | 3.67 | 3.73 | 3.77 |
| Refrigerating Capacity | kJ/m³ | 4066 | 4260 | 4612 | 4905 | 5169 | 5416 | 5524 |
| COP in Comparison | % (relative to R410A) | 99.2 | 98.0 | 96.4 | 96.2 | 97.1 | 98.7 | 99.6 |
| Refrigerating Capacity in Comparison | % (relative to R410A) | 72.2 | 75.7 | 81.9 | 87.1 | 91.8 | 96.2 | 98.1 |
| Discharge Temperature | °C | 63.4 | 65.0 | 67.9 | 70.6 | 73.6 | 76.9 | 78.5 |
| Discharge Pressure | MPa | 2.1 | 2.2 | 2.4 | 2.5 | 2.6 | 2.7 | 2.7 |
| Evaporation Pressure | MPa | 0.7 | 0.8 | 0.9 | 0.9 | 0.9 | 0.9 | 1.0 |
| Condensation Glide | K | 0.1 | 0.6 | 0.6 | 0.3 | 0.1 | 0.0 | 0.0 |
| Evaporation Glide | K | 0.1 | 0.5 | 0.6 | 0.2 | 0.0 | 0.0 | 0.0 |
| Boiling Point | °C | -47.6 | -49.8 | -52.2 | -53.2 | -53.5 | -53.3 | -53.1 |
| Density (Intake) | g/cm³ | 31.0 | 32.1 | 33.6 | 33.7 | 32.7 | 31.1 | 30.3 |

### Reference Example 3

Fig. 1 illustrates an example of the process for separating HFO-1132(E) and HFC-143. This process is an example of the process of separation by distillation that uses the properties of an azeotropic composition containing HFO-1132(E) and HFC-143a.

In S11, a starting composition containing HFO-1132(E) and HFC-143a is fed into a distillation column C1. In S12, an azeotropic composition containing HFO-1132(E) and HFC-143a flows out. In S13, HFO-1132(E), together with HFC-143a the concentration of which is lower than that in the starting composition, is obtained. Table 3 illustrates the results of separation by distillation.

**Table 3**

| | Flow Rate (kg/hr) | | |
|---|---|---|---|
| | S11 | S12 | S13 |
| HFC-143a | 0.06 | 0.06 | 0.00 |
| HFO-1132 (E) | 9.94 | 0.52 | 9.42 |

The operating pressure is 0.2 MPa, and the top temperature is -38°C. The number of stages of the distillation column is 50, and the number of feed stages is 35. A composition of HFC-143a and HFO-1132(E) with an azeotropic formulation flows out from the top, and HFO-1132(E), together with HFC-143a the concentration of which is decreased flows out from the bottom.

This process enables the construction of a purification process for HFO-1132(E) with minimized loss.

## Claims

1. Use of a composition comprising a refrigerant as an alternative to R410A, wherein
the refrigerant is azeotropic or azeotrope-like and comprises trans-1,2-difluoroethylene (HFO-1132(E)) and 1,1,1-trifluoroethane (HFC-143a), and
the proportion of HFO-1132(E) is 80 mass% or more and the proportion of HFC-143a is 20 mass% or less based on the total amount of HFO-1132(E) and HFC-143a in the refrigerant.

2. The use according to claim 1, wherein the refrigerant further comprises an additional refrigerant, and the additional refrigerant is at least one refrigerant selected from the group consisting of 1,1,2-trifluoroethylene (HFO-1123), 1,1-difluoromethane, fluoroethane (HFC-152a), 1,1,2-trifluoroethane (HFC-143), 2-chloro-1,1,1-trifluoroethane (CFC-133a), 1-chloro-1,1,2-trifluoroethane (HCFC-133b), 2,2-dichloro-1,1,1-trifluoroethane (HCFC-123), and 1,2-difluoroethane (HFC-152).

3. The use according to claim 1 or 2, wherein the proportion of the total amount of the additional refrigerant in the refrigerant is more than 0 mass% and 1 mass% or less based on the total amount of HFO-1132(E), HFC-143a, and the total amount of the additional refrigerant.

## Patentansprüche

1. Verwendung einer Zusammensetzung, die ein Kältemittel als Alternative zu R410A umfasst, wobei
das Kältemittel azeotrop oder azeotrop-ähnlich ist und trans-1,2-Difluorethylen (HFO-1132(E)) und 1,1,1-Trifluorethan (HFC-143a) umfasst, und
der Anteil an HFO-1132(E) 80 Massenprozent oder mehr und der Anteil an HFC-143a 20 Massenprozent oder weniger beträgt, bezogen auf die Gesamtmenge an HFO-1132(E) und HFC-143a im Kältemittel.

2. Verwendung gemäß Anspruch 1, wobei das Kältemittel ferner ein zusätzliches Kältemittel umfasst und das zusätzliche Kältemittel mindestens ein Kältemittel ist, ausgewählt aus der Gruppe bestehend aus 1,1,2-Trifluorethylen (HFO-1123), 1,1-Difluormethan, Fluorethan (HFC-152a), 1,1,2-Trifluorethan (HFC-143), 2-Chlor-1,1,1-trifluorethan (CFC-133a), 1-Chlor-1,1,2-trifluorethan (HCFC-133b), 2,2-Dichlor-1,1,1-trifluorethan (HCFC-123) und 1,2-Difluorethan (HFC-152).

3. Verwendung gemäß Anspruch 1 oder 2, wobei der Anteil der Gesamtmenge des zusätzlichen Kältemittels am Kältemittel mehr als 0 Massenprozent und 1 Massenprozent oder weniger beträgt, bezogen auf die Gesamtmenge von HFO-1132(E), HFC-143a und die Gesamtmenge des zusätzlichen Kältemittels.

## Revendications

1. Utilisation d'une composition comprenant un fluide frigorigène comme fluide frigorigène alternatif pour du R410A, dans laquelle
le fluide frigorigène est azéotropique ou quasi-azéotropique et comprend *trans-*1,2-difluoroéthylène (HFO-1132(E)) et 1,1,1-trifluoroéthane (HFC-143a), et
la proportion de HFO-1132(E) est de 80 % en masse ou plus et la proportion de HFC-143a est de 20 % en masse ou moins sur la base de la quantité totale de HFO-1132(E) et de HFC-143a dans le fluide frigorigène.

2. Utilisation selon la revendication 1, dans laquelle le fluide frigorigène comprend en outre un fluide frigorigène additionnel, et le fluide frigorigène additionnel est au moins un fluide frigorigène sélectionné dans le groupe consistant en 1,1,2-trifluoroéthylène (HFO-1123), difluorométhane, fluoroéthane (HFC-152a), 1,1,2-trifluoroéthane (HFC-143), 2-chloro-1,1,1-trifluoroéthane (CFC-133a), 1-chloro-1,1,2-trifluoroéthane (HCFC-133b), 2,2-dichloro-1,1,1-trifluoroéthane (HCFC-123), et 1,2-difluoroéthane (HFC-152).

3. Utilisation selon la revendication 1 ou la revendication 2, dans laquelle la proportion de la quantité totale du fluide frigorigène additionnel dans le fluide frigorigène est supérieure à 0 % en masse et inférieure ou égale à 1 % en masse sur la base de la quantité totale de HFO-1132(E), de HFC-143a, et de la quantité totale du fluide frigorigène additionnel.
